# EUROPÄISCHE PATENTSCHRIFT

(11) **EP 3 600 782 B1**
(45) Veröffentlichungstag und Bekanntmachung des Hinweises auf die Patenterteilung: **12.05.2021**
(21) Anmeldenummer: 18713222.0
(22) Anmeldetag: 23.03.2018
(51) Int. Cl.: B25F 5/00

(54) **WERKZEUGMASCHINE**
MACHINE TOOL
MACHINE-OUTIL

(30) Priorität: 30.03.2017 DE 102017205430
(43) Veröffentlichungstag der Anmeldung: 05.02.2020
(73) Patentinhaber: Robert Bosch GmbH, 70442 Stuttgart (DE)
(72) Erfinder: SCHADOW, Joachim, 70563 Stuttgart (DE); STOCK, Joern, 70711 Leinfelden-Echterdingen (DE); OSSWALD, Alexander, 70599 Stuttgart (DE); ESENWEIN, Florian, 70771 Leinfelden-Echterdingen (DE)
(86) Internationale Anmeldenummer: PCT/EP2018/057454
(87) Internationale Veröffentlichungsnummer: WO 2018/177932

(56) Entgegenhaltungen:
- EP-A1- 3 112 089
- WO-A1-2016/146705
- DE-A1-102013 201 359
- DE-A1-102014 209 032
- DE-A1-102015 206 608
- DE-A1-102015 214 121
- US-A1- 2003 107 470
- US-A1- 2014 231 113

## Beschreibung

Die Erfindung bezieht sich auf eine Werkzeugmaschine, insbesondere eine Handwerkzeugmaschine, nach dem Oberbegriff des unabhängigen Anspruchs.

### Stand der Technik

Die DE 10 2013 202 832 A1 offenbart eine elektrische Handwerkzeugmaschine mit einem Gerätegehäuse, einer gerätegehäuseseitigen Koppeleinrichtung zum Befestigen eines Zusatzhandgriffs am Gerätegehäuse und einer Detektionseinrichtung zum Detektieren des Zusatzhandgriffs am Gerätegehäuse und/oder des Festhaltens des am Gerätegehäuse befestigten Zusatzhangriffs durch einen Anwender, umfassend einen elektrischen Antriebsmotor und eine Steuereinrichtung zum Steuern des Antriebsmotors mit einer Auswerteeinrichtung zum Auswerten des aktuellen Betriebszustands der elektrischen Handwerkzeugmaschine und einer Aktivierungseinrichtung zum Aktivieren einer Schutzfunktion, wenn die Auswerteeinrichtung das Auftreten eines kritischen Betriebsfalls erkennt. Die Steuereinrichtung ist ausgebildet, die Auswertung des aktuellen Betriebszustands und/oder die Aktivierung der Schutzfunktion in Abhängigkeit von der Detektion des Zusatzhandgriffs am Gerätegehäuse und/oder des Festhaltens des Zusatzhandgriffs durch den Anwender durchzuführen. Andere Beispiele sind aus der EP3112089A1, DE102015206608A1, DE102013201359A1, DE102015214121A1 oder US2014231113A1 bekannt.

### Offenbarung der Erfindung

Der Erfindung liegt die Aufgabe zugrunde, mit einfachen Maßnahmen die Werkzeugmaschine zu verbessern.

Die Aufgabe wird gelöst mit einer Werkzeugmaschine, insbesondere einer Handwerkzeugmaschine, wie in Anspruch 1 dargestellt, mit einer Antriebseinheit zum Antrieb eines mit der Werkzeugmaschine betreibar aufnehmbaren Einsatzwerkzeugs, mit einer Steuer- oder Regeleinheit zur Steuerung oder Regelung der Antriebseinheit.

Erfindungsgemäß weist die Werkzeugmaschine zumindest eine erste Sensoreinheit mit einem Bildsensor auf, wobei die Sensoreinheit dazu eingerichtet ist, bedienerspezifische Daten zu sensieren und mittels der Steuer- oder Regeleinheit derart zu verarbeiten, dass die Antriebseinheit in Abhängigkeit der sensierten Daten gesteuert oder geregelt wird.

Die erfindungsgemäße Werkzeugmaschine ermöglicht mehrere Vorteile, welche die Handhabung der Werkzeugmaschine verbessern, indem der Bediener beobachtet wird. Dabei werden bedienerspezifische Daten sensiert, um die Antriebseinheit zu steuern oder zu regeln. Hierdurch kann beispielsweise die Sicherheit des Bedieners der Werkzeugmaschine erhöht werden, indem überprüft wird, ob beispielsweise sicherheitsrelevante Zubehöreinrichtungen an dem Bediener und/oder der Werkzeugmaschine verwendet werden bzw. vorhanden sind.

Mittels der erfindungsgemäßen Sensoreinheit können Daten des Bedieners, insbesondere berührungslos, sensiert werden. Mittels des Bildsensors kann der Bediener der Werkzeugmaschine zuverlässig und insbesondere durchgängig oder in zeitlichen Abständen beobachtet werden. Die von dem Bildsensor sensierten Daten können in Form von zweidimensionalen oder dreidimensionalen Abbildungen der Umwelt bzw. der unmittelbaren Umgebung um die Werkzeugmaschine ausgewertet und in Abhängigkeit der ausgewerteten Daten die Antriebseinheit mittels der Steuer- oder Regeleinheit gesteuert oder geregelt werden.

Mittels der erfindungsgemäßen Werkzeugmaschine kann überprüft werden, ob die Werkzeugmaschine in einer geeigneten Arbeitsumgebung betrieben wird, sodass beispielsweise etwaige Lichtverhältnisse der Arbeitsumgebung überprüft und die Antriebseinheit entsprechend gesteuert oder geregelt wird.

Unter einer "Sensoreinheit" soll eine Umgebungssensoreinheit verstanden werden, welche zumindest ein Umgebungssensorelement zu einer Erfassung von zumindest einer Umgebungskenngröße aufweist, die eine die Werkzeugmaschine umgebende Umwelt definiert, eine Einwirkung der Werkzeugmaschine auf die umgebende Umwelt definiert und/oder eine Positionierung der Werkzeugmaschine relativ zur umgebenden Umwelt definiert. Hierbei ist die Umgebungssensoreinheit vorzugsweise dazu eingerichtet, zumindest eine, insbesondere zweidimensionale und/oder eine dreidimensionale, Abbildung einer Umgebung aufzunehmen und insbesondere mittels einer Auswerteeinheit beispielsweise in Form eines Prozessors auszuwerten. Dabei kann die Steuer- oder Regeleinheit die Auswerteeinheit umfassen. Die Steuer- oder Regeleinheit ist zumindest vorzugsweise dazu eingerichtet, die Antriebseinheit in Abhängigkeit von der zumindest einen mittels einer Antriebseinheitssensoreinheit erfassten Antriebseinheitskenngröße und in Abhängigkeit von der zumindest einen mittels der Umgebungssensoreinheit erfassten Umgebungskenngröße zu steuern oder zu regeln. Zusätzlich ist die Steuer- oder Regeleinheit bevorzugt zumindest dazu vorgesehen, eine Information in Abhängigkeit von der zumindest einen mittels der Antriebseinheitssensoreinheit erfassten Antriebseinheitskenngröße und in Abhängigkeit von der zumindest einen mittels der Umgebungssensoreinheit erfassten Umgebungskenngröße an einen Bediener auszugeben. Bevorzugt ist zumindest eine Antriebseinheitskennlinie, eine maximale Drehzahl, eine minimale Drehzahl, ein maximales Drehmoment und/oder ein minimales Drehmoment der Antriebseinheit mittels der Steuer- oder Regeleinheit steuerbar oder regelbar. Dabei kann beispielsweise die Antriebseinheit deaktiviert werden oder ein Antriebseinheitskenngröße verringert oder erhöht werden.

Die Sensoreinheit kann eine Objektiveinheit aufweisen. Die Objektiveinheit kann ein, insbesondere als eine Sammellinse ausgebildetes, optisches Objektiv umfassen, welches insbesondere dazu vorgesehen ist, einen Blickwinkel bzw. einen Bildwinkel und/oder einen Brennpunkt einzustellen und/oder zu verändern, vorzugsweise zu vergrößern oder zu verkleinern. Die Objektiveinheit kann dabei einen Blickwinkel von mindestens 120°, insbesondere mindestens 180°, vorzugsweise mindestens 270°, bevorzugt mindestens 330°, wie beispielsweise 360°, ermöglichen, sodass die Sensoreinheit einen weiten Blickwinkelbereich sensieren kann. Die Objektiveinheit ist dazu vorgesehen, Licht und/oder Infrarotstrahlungen zumindest teilweise zu bündeln und/oder zu streuen, um eine Abbildung der Umwelt auf dem Bildsensor darzustellen.

Unter einer "Steuer- oder Regeleinheit" soll insbesondere eine Einheit mit einer Prozessoreinheit und mit einer Speichereinheit sowie mit einem in der Speichereinheit gespeicherten Betriebsprogramm verstanden werden. Die Speichereinheit kann dazu eingerichtet sein, die von der Sensoreinheit sensierten Daten zu speichern. Unter "eingerichtet" soll insbesondere speziell programmiert, speziell ausgelegt und/oder speziell ausgestattet verstanden werden. Darunter, dass ein Element und/oder eine Einheit zu einer bestimmten Funktion vorgesehen sind/ist, soll insbesondere verstanden werden, dass das Element und/oder die Einheit diese bestimmte Funktion in zumindest einem Anwendungs- und/oder Betriebszustand erfüllen/erfüllt und/oder ausführen/ausführt.

Unter einer "Antriebseinheit" soll in diesem Zusammenhang insbesondere eine Einheit verstanden werden, die dazu vorgesehen ist, zumindest ein Antriebsmoment zu erzeugen und zur Weiterleitung insbesondere an ein Einsatzwerkzeug zur Verfügung zu stellen. Vorteilhaft weist die Werkzeugmaschine die Antriebseinheit auf. Besonders vorteilhaft weist die Antriebseinheit zumindest einen Elektromotor auf. Vorzugsweise ist die Antriebseinheit dazu eingerichtet, zumindest ein Einsatzwerkzeug der Werkzeugmaschine anzutreiben und/oder in Bewegung zu versetzen.

Bei der Werkzeugmaschine handelt es sich vorzugsweise um eine handgehaltene, eine handgeführte, eine handbetriebene und/oder eine autonom arbeitende Werkzeugmaschine, welche als nicht-stationäre Werkzeugmaschine ausgebildet ist und beispielsweise für den Heimwerkergebrauch geeignet ist. Prinzipiell eignen sich als Werkzeugmaschinen nicht-stationäre Werkzeugmaschine wie beispielsweise eine Handkreissäge entsprechend der Anmeldung DE 3740200 A1 oder wie beispielsweise ein rückentragbares Freischneidegerät entsprechend der Anmeldung DE 19674764 A1. Ferner eignen sich auch Gartenwerkzeugmaschinen, insbesondere Rasenmäher. Auch kommt eine Verwendung bei Handwerkzeugmaschinen, insbesondere Bohrwerkzeugmaschinen, Schraubendrehwerkzeugmaschinen, Schleifwerkzeugmaschinen oder eine Trennwerkzeugmaschinen, in Betracht.

Unter einem "Bildsensor" soll eine Vorrichtung zur Aufnahme einer Abbildung einer Umweltkenngröße aus Licht und/oder Infrarotstrahlungen verstanden werden, welche insbesondere auf elektrischem und/oder auf mechanischem Weg erfolgen können. Die Bildsensoren können auf Halbleiterbasis aufgebaut sein. Die Bildsensoren können dabei elektromagnetische Wellen mit einer Wellenlänge, von beispielsweise 380 bis 750 nm (sichtbares Licht), 780 bis 1400 nm (nahes Infrarot), 1400 bis 3000 nm (nahes Infrarot) und/oder 3000 bis 50000 nm (mittleres Infrarot), sensieren und zweckmäßigerweise Daten der jeweiligen Wellenlängenbereiche auswerten. Es versteht sich, dass ein Fachmann etwaige Vorzüge von sensierten Daten aus den jeweiligen Wellenlängenbereichen kennt und diese zweckmäßig für eine Auswertung der sensierten Daten verwendet.

Unter "bedienerspezifisch" soll den Bediener betreffend verstanden werden, sodass insbesondere etwaige sensierte Daten des Bedieners oder der unmittelbaren Umgebung des Bedieners verwendet werden, um die Sicherheit der Werkzeugmaschine zu erhöhen.

Die Unteransprüche geben zweckmäßige Weiterbildungen der erfindungsgemäßen Werkzeugmaschine an.

Es kann zweckmäßig sein, dass die zumindest eine erste Sensoreinheit dazu eingerichtet ist, Vitaldaten, insbesondere eine Herzfrequenz und/oder einen Ermüdungszustand, eines Bedieners zu erfassen. Durch das Sensieren der Herzfrequenz kann beispielsweise erfasst werden, ob der Bediener eine ausreichend hohe Herzfrequenz hat und/oder inwieweit diese sich im Betrieb der Werkzeugmaschine ändert, insbesondere erhöht oder verringert. Falls die Herzfrequenz zu gering ist oder gar überhöht ist, kann beispielsweise die Antriebseinheit abgeschaltet werden, sodass der Bediener die Werkzeugmaschine vorübergehend nicht bedienen kann. Auch kann zum Beispiel ein zu schnelles Ansteigen der Herzfrequenz darauf hindeuten, dass der Bediener möglicherweise eine zu schwere Arbeit ausführt, sodass beispielsweise die Antriebseinheit entsprechend gesteuert oder geregelt wird. Hierdurch wird sichergestellt, dass der Bediener bei längerem Betrieb der Werkzeugmaschine leistungsfähig genug für die ihm bevorstehende Arbeit ist.

Die zumindest eine erste Sensoreinheit kann die Haut des Bedieners sensieren und in Abhängigkeit von einer sich ändernden Hautfarbe die Herzfrequenz bestimmen. Die Hautfarbe ist bei einem Herzschlag stärker durchblutet und weist somit in der Regel eine sattere Farbe auf. Zwischen zwei Herzschlägen ist die Haut schwächer durchblutet und weist in der Regel eine blassere Hautfarbe auf. Sensiert die erste Sensoreinheit die Hautunterschiede in einem bestimmten Zeitintervall, so kann in Abhängigkeit der Änderung der Hautfarbe auf die Herzfrequenz des Bedieners geschlossen werden.

Ferner kann es zweckmäßig sein, dass die zumindest eine erste Sensoreinheit dazu eingerichtet ist, zumindest eine Zubehöreinrichtung des Bedieners, insbesondere eine Schutzbrille und/oder einen Handschuh und/oder einen Sicherheitsschuh, zu erfassen. Insbesondere kann die zumindest eine erste Sensoreinheit dazu eingerichtet sein zu überprüfen, ob der Bediener die Zubehöreinrichtung trägt. Hierdurch könnte auf besonders einfache Weise überprüft werden, ob der Bediener entsprechende Zubehöreinrichtungen verwendet bzw. trägt. In Abhängigkeit eines Vorhandenseins der zumindest einen Zubehöreinrichtung zum Schutz des Bedieners könnte die Werkzeugmaschine bzw. die Antriebseinheit eine Verwendung der Werkzeugmaschine verhindern. Beispielsweise kann die Werkzeugmaschine auch ohne eine Zubehörvorrichtung in Betrieb genommen werden, falls bei der Bearbeitung des Werkstücks lediglich wenig Staub oder wenig Späne in der Umgebung umhergewirbelt werden.

Weiterhin kann es zweckmäßig sein, dass die zumindest eine erste Sensoreinheit dazu eingerichtet ist, zumindest ein Auge oder eine Lidbewegung eines Auges des Bedieners zu überwachen, um einen Ermüdungszustand oder eine Verletzung des Auges zu erkennen. Hierdurch kann auf besonders effektive Weise eine Übermüdung des Bedieners erkannt werden. Auch kann erkannt werden, ob der Bediener sich möglicherweise am Auge verletzt hat, indem der Bediener die Lider zumindest eines Auges schließt bzw. das zumindest eine Auge mit zumindest einer Hand verdeckt ohne die Werkzeugmaschine auszuschalten.

Insbesondere kann die erste Sensoreinheit dazu eingerichtet sein, eine Schwenkbewegung bzw. eine Verdrehbewegung des Kopfs des Bedieners zu sensieren. Hierbei kann beispielsweise ein Augenabstand beider Augen des Bedieners oder eine Formänderung zumindest einer Augenkontur eines, insbesondere jedes, Auges des Bedieners erfasst werden. Hierdurch kann beispielsweise ein Augenabstand beider Augen des Bedieners oder eine Formänderung zumindest einer Augenkontur des Bedieners derart erfasst werden, dass erkannt wird, ob der Bediener eine Blickrichtung auf das zu bearbeitende Werkstück des Arbeitsbereichs richtet. Hierdurch kann auf den Aufmerksamkeitszustand oder den Ermüdungszustand des Bedieners im Betrieb der Werkzeugmaschine geschlossen werden. Neben einer Ermüdungserkennung können auch weitere Aspekte des Bedieners und/oder der Arbeitsumgebung analysiert werden, wie zum Beispiel eine Pupillengröße zumindest eines Auges des Bedieners, insbesondere in Kombination mit der Umgebungshelligkeit des Arbeitsbereichs. Beispielsweise kann auf mangelhafte Lichtverhältnisse des Arbeitsbereichs geschlossen werden, wenn die Pupillengröße bei mangelnden Lichtverhältnissen gegenüber der Pupillengröße bei guten Lichtverhältnissen verhältnismäßig groß sind.

Alternativ oder zusätzlich kann die erste Sensoreinheit dazu eingerichtet sein, eine Schwenkbewegung zumindest einer Pupille eines, insbesondere jedes, Auges des Bedieners zu sensieren. Hierbei kann beispielsweise ein Pupillenabstand beider Pupillen des Bedieners oder eine Formänderung zumindest einer, insbesondere jeder, Pupillenkontur des Bedieners derart erfasst werden, dass erkennt wird, ob der Bediener eine Blickrichtung auf das zu bearbeitende Werkstück des Arbeitsbereichs richtet. Die erste Sensoreinheit kann derart an der Werkzeugmaschine angeordnet und ausgerichtet sein, dass die Pupillenkontur eines sich auf das zu bearbeitende Werkstück gerichteten Auges des Bedieners beispielsweise im Wesentlichen kreisrund ist und dass die Pupillenkontur eines sich von dem zu bearbeitenden Werkstück weg gerichteten Auges des Bedieners beispielsweise im Wesentlichen ellipsenförmig ist. Prinzipiell kommen aber auch andere Messmethoden in Betracht, mit welchen mittels einer Sensoreinheit mit einem Bildsensor auf geeignete Weise eine Blickrichtung des Bedieners sensiert werden kann.

Zur Sensierung entsprechender bedienerspezifischen Daten, wie beispielsweise einer Formänderung einer Pupille, kann die Sensoreinheit ortsfest an der Werkzeugmaschine derart angeordnet sein, dass in Abhängigkeit einer Relativbewegung bedienerspezifischer Daten, wie beispielsweise der Pupillengröße, gegenüber der Sensoreinheit auf eine Änderung der bedienerspezifischen Daten geschlossen werden kann.

Unter "ellipsenförmig" soll eine geometrische Form einer Ellipse mit einer Hauptachse, welche einen großen Durchmesser der Ellipse definiert, und einer orthogonal zu der Hauptachse verlaufende Nebenachse, welche einen kleinen Durchmesser der Ellipse definiert, verstanden werden. Die Hauptachse definiert die maximale Erstreckung der Ellipse. Die Nebenachse definiert die minimale Erstreckung der Ellipse. Unter "im Wesentlichen kreisrundförmig" soll eine geometrische Form eines Kreises mit einem konstanten Durchmesser verstanden werden, welche gegenüber einer elliptischen Form insbesondere eine maximale Abweichung von maximal 10 %, insbesondere maximal 5 %, vorzugsweise maximal 2 %, und entsprechend eine Hauptachse des Kreises gegenüber einer Nebenachse des Kreises eine maximale Längenabweichung von 10 %, insbesondere von 5 %, vorzugsweise von 2 %, aufweist.

Unter einer "Augenkontur" soll insbesondere eine Kontur verstanden werden, welche das Auge des Bedieners definiert und insbesondere durch beispielsweise die Augenlieder begrenzt ist. Unter einer Pupillenkontur soll insbesondere eine Kontur verstanden werden, welche die Pupille des Auges des Bedieners definiert und insbesondere durch beispielsweise die Regenbogenhaut des Auges begrenzt ist.

Des Weiteren kann es zweckmäßig sein, dass die zumindest eine erste Sensoreinheit dazu eingerichtet ist, ein Gesicht des Bedieners zu sensieren und insbesondere zu erkennen, ob der Bediener handhabungsberechtigt ist. Dabei kann beispielsweise ein Gesicht oder einzelne, insbesondere markante, Gesichtspartien des Bedieners mittels der Sensoreinheit sensiert und in einer Speichereinheit abgespeichert werden. Vor und/oder bei einem Betrieb der Werkzeugmaschine kann die Sensoreinheit das Gesicht oder einzelne Gesichtspartien des Bedieners sensieren und mittels der gespeicherten Daten abgleichen, um eine Bedienung der Werkzeugmaschine freizugeben oder zu sperren.

Hierdurch kann auf besonders einfache Weise ein Bedienerkreis von Bedienern festgelegt werden, welcher berechtigt ist, die Werkzeugmaschine zu betätigen bzw. zu betreiben. Hierdurch kann sowohl ein zuverlässiger Diebstahlschutz als auch eine zuverlässige Kindersicherung erreicht werden, indem die nicht dem Bedienerkreises zugehörigen Bediener von einer Bedienung der Werkzeugmaschine ausgeschlossen werden.

Es wird vorgeschlagen, dass die zumindest eine erste Sensoreinheit dazu eingerichtet ist, einen Arbeitsbereich zu beobachten, um die Antriebeinheit in Abhängigkeit eines sich in dem Arbeitsbereich befindlichen Körperteils eines Bedieners oder einer sich in dem Arbeitsbereich befindlichen Person zu steuern oder zu regeln. Hierdurch kann auf besonders einfache Weise festgestellt werden, ob sich ein Teil eines Bedieners oder eine weitere Person im Arbeitsbereich der Werkzeugmaschine befindet.

Es wird ferner vorgeschlagen, dass die zumindest eine erste Sensoreinheit dazu eingerichtet ist, einen Istzustand der Werkzeugmaschine in zumindest einem Arbeitsschritt vor und/oder während des Betriebszustands der Antriebseinheit zu sensieren und mit einem Sollzustand abzugleichen und in Abhängigkeit einer Abweichung vom Istzustand gegenüber dem Sollzustand die Antriebseinheit zu steuern oder zu regeln. Unter einem "Arbeitsschritt" soll ein Bearbeitungsschritt bei einer Bearbeitung eines Werkstücks verstanden werden, in welchem die Antriebseinheit eingeschaltet und betrieben ist. Vor dem Arbeitsschritt soll bedeuten, dass die Antriebseinheit noch nicht in einem Betriebszustand ist, der Bediener die Werkzeugmaschine jedoch bereits in einer Betriebsstellung hält oder die Werkzeugmaschine mit zumindest einer Hand greift oder einen Schalter der Werkzeugmaschine bereits derart betätigt, dass die Antriebseinheit nicht in den Betriebszustand versetzt wird.

Es wird weiter vorgeschlagen, dass die zumindest eine erste Sensoreinheit dazu eingerichtet ist, zu erkennen, ob der Bediener die Werkzeugmaschine betätigt. Unter "betätigt" soll verstanden werden, dass der Bediener die Werkzeugmaschine in einen Betriebszustand versetzt und vorzugsweise zumindest mit einer Hand die Werkzeugmaschine hält. Dabei kann beispielsweise der Blickwinkel des Bildsensors derart ausgelegt sein, dass sensiert wird, ob eine Hand des Bedieners an einem Griffbereich der Werkzeugmaschine angeordnet ist oder diesen umgreift. Hierdurch kann auf besonders einfache Weise erkannt werden, ob der Bediener die Handwerkzeugmaschine noch hält oder umgreift. Dadurch kann eine weitere oder eine zusätzliche Vorrichtung bereitgestellt werden, welche beispielsweise zusätzlich zu den bestehenden Vorrichtungen zur Erkennung eines unzulässigen Betriebszustands, wie beispielsweise bei einem versehentlichen Fallenlassen der Werkzeugmaschine, die Antriebseinheit deaktiviert. Hierdurch kann zuverlässig eine Widereinschaltsperre erreicht werden. Insbesondere kann die Antriebseinheit bei einer fallengelassenen Werkzeugmaschine bereits im freien Fall der Werkzeugmaschine deaktiviert werden.

Es kann zweckmäßig sein, dass die Werkzeugmaschine zumindest eine zweite Sensoreinheit aufweist, welche zu der zumindest einen ersten Sensoreinheit beabstandet ist, wobei die erste Sensoreinheit auf einen Bediener der Werkzeugmaschine ausgerichtet ist und die zweite Sensoreinheit auf den Arbeitsbereich ausgerichtet ist. Insbesondere kann die zumindest eine zweite Sensoreinheit, dazu eingerichtet sein, einen Arbeitsbereich zu beobachten, um die Antriebseinheit in Abhängigkeit eines sich in dem Arbeitsbereich befindlichen Körperteils eines Bedieners oder einer sich in dem Arbeitsbereich befindlichen Person zu steuern oder zu regeln. Hierdurch kann sowohl der Bediener als auch der Arbeitsbereich sensiert werden. Hierdurch kann beispielsweise erkannt werden, ob der Arbeitsbereich unzulässig für eine Bearbeitung ist, indem beispielsweise sensiert wird, ob der Arbeitsbereich ausreichend beleuchtet ist und/oder ob der Bediener im Betrieb der Werkzeugmaschine auf den Arbeitsbereich schaut.

Durch eine Beobachtung des Arbeitsbereichs kann eine Fehlbedienung der Werkzeugmaschine verhindert werden, indem beispielsweise erkannt wird, dass ein Bohrwerkzeug nicht richtig eingespannt ist oder eine Hand des Bedieners oder einer anderen Person zu nahe an ein sich im Betrieb befindliches Sägeblatt kommt.

Insbesondere bei einem Messgerät wie einem Laser-Entfernungsmesser kann der Laserstrahl deaktiviert werden, sobald ein Gesicht einer Person im Zielbereich des Lasers sensiert wird. Vorzugsweise bei einem Rasenmäher können Hindernisse wie beispielsweise Personen, insbesondere Kinder, oder Tiere, insbesondere Haustiere, sensiert und schnell und eindeutig identifiziert werden, um die Werkzeugmaschine zu deaktivieren oder die Hindernisse zu umfahren.

Ferner kann es zweckmäßig sein, dass die erste und/oder die zweite Sensoreinheit Daten im Infrarotstrahlungsbereich sensiert. Hierdurch können besonders einfach Gefahrenquellen, wie beispielsweise Menschen oder Tiere erkannt werden. Insbesondere kann eine Zubehörvorrichtung, wie beispielsweise eine Schutzbrille oder ein Handschuh, einen Reflektor, wie beispielsweise einen Rückstrahler, aufweisen, welcher insbesondere dazu eingerichtet ist, Infrarotstrahlung in beispielsweise einem Wellenlängenbereich von 780 nm bis 1400 nm und/oder 1400 nm bis 3000 nm und/oder von 3000 nm bis 50000 nm zu reflektieren. Hierdurch kann eine Erkennung der Zubehörvorrichtung mittels der ersten oder der zweiten Sensoreinheit erleichtert werden. Der Reflektor soll dabei einfallende Strahlung einer bestimmten Wellenlänge, wie beispielsweise einer Wellenlänge im Infrarotstrahlungsbereich, reflektieren bzw. zurückstrahlen. Die Sensoreinheit kann die reflektierte Strahlung entsprechend aufnehmen, und eine Auswerteeinheit kann dieses dann auswerten.

Vorzugsweise können die Werkzeugmaschine und/oder die erste Sensoreinheit ein Infrarotstrahlungsmittel aufweisen, welches dazu vorgesehen ist, Infrarotstrahlung auszusenden. Insbesondere kann die von dem Infrarotstrahlungsmittel auszusenden Infrarotstrahlung am Reflektor reflektiert werden. Vorzugsweise kann die vom Reflektor reflektierte Strahlung mittels des Bildsensors der ersten oder der zweiten Sensoreinheit sensiert werden. Weiterhin kann es zweckmäßig sein, dass die Werkzeugmaschine zumindest eine Kommunikationseinheit zu einer Kommunikation mit zumindest einer externen Einheit zu einem Austausch von elektronischen Daten zumindest zu einer Steuerung oder Regelung der Antriebseinheit aufweist. Die Kommunikationseinheit ist vorzugsweise als kabellose Kommunikationseinheit ausgebildet. Hierbei kann die Kommunikationseinheit als WLAN-Kommunikationseinheit, als Bluetooth-Kommunikationseinheit, als Funk-Kommunikationseinheit, als RFID-Kommunikationseinheit, als NFC-Einheit, als Infrarot-Kommunikationseinheit, als Mobilfunknetz-Kommunikationseinheit o. dgl. ausgebildet sein. Besonders bevorzugt ist die Steuer- oder Regeleinheit dazu vorgesehen, die Antriebseinheit und/oder Sicherheitsfunktionen in Abhängigkeit der zumindest einen mittels der Umgebungssensoreinheit erfassten Umgebungskenngröße und in Abhängigkeit von mittels der Kommunikationseinheit an die Steuer- oder Regeleinheit übertragenen elektronischen Daten zu steuern oder zu regeln. Besonders bevorzugt ist die Kommunikationseinheit zu einer bidirektionalen Datenübertragung vorgesehen. In einer alternativen Ausgestaltung ist die Kommunikationseinheit als kabelgebundene Kommunikationseinheit ausgebildet, wie beispielsweise als LAN-Kommunikationseinheit, als USB-Kommunikationseinheit o. dgl. Die externe Einheit ist vorzugsweise als Smartphone ausgebildet, das eine App zu einer Kommunikation mit der Kommunikationseinheit aufweist. Es ist jedoch auch denkbar, dass die externe Einheit als externe, transportable Bedieneinheit, als fest installierte Bedieneinheit an einem Arbeitsplatz eines Bedieners, als fest in einem Raum installierte Synchronisationseinheit eines Einsatzortes, die von einer Zentrale gesteuert werden kann, wie beispielsweise infolge von Firmenvorgaben/Sicherheitsbestimmungen, als Bedienerkörperkenngrößenüberwachungseinheit, als externe Sensoreinheit oder als weitere, einem Fachmann als sinnvoll erscheinende zentrale oder dezentrale Bedieneinheit, Eingabestation und/oder zentrales oder dezentrales Terminal ausgebildet ist. Es kann somit vorteilhaft eine Synchronisation von elektronischen Daten ermöglicht werden. Wird beispielsweise die Werkzeugmaschine in einem Synchronisationsmodus in Betrieb genommen, beispielsweise durch ein Einstecken einer Akkumulatorvorrichtung, bei einem Einstecken eines Stromversorgungskabels oder durch eine Aktivierung durch einen Bediener, wird zumindest teilweise automatisch eine Verbindung zwischen der Kommunikationseinheit und der externen Einheit aufgebaut. In der externen Einheit hinterlegte Einstellungen sind somit vorzugsweise direkt auf die Werkzeugmaschine übertragbar. Hierbei kann es sich um individuelle Einstellungen eines Bedieners, wie beispielsweise ein gewünschtes, schnelles Hochfahren auf eine eingestellte Drehzahl und eine maximale Leistung und/oder um Vorgaben einer Firma, wie beispielsweise eine Einhaltung einer Sicherheitsfunktion in einem vorgegebenen Bereich eines Firmengeländes oder eines Einsatzorts usw. handeln.

Zudem sind elektronische Daten mittels der Kommunikationseinheit an die externe Einheit übertragbar. Hierbei ist beispielsweise eine Vibrationsbelastung eines Bedieners zu einer Kontrolle einer Einhaltung einer Belastungsgrenze und/oder eine eventuelle Bezahlung von Zulagen und/oder eine Laufzeit und eine Last zu einer Beurteilung einer Werkzeugmaschinenauslastung an eine Firmenzentrale o. dgl. übertragbar. Es ist zudem denkbar, dass die externe Einheit ein Vorhandensein einer Sicherheitsausrüstung und/oder einer geeigneten Arbeitskleidung überprüft, wie beispielsweise mittels RFID-Kennung usw., wobei die externe Einheit in Abhängigkeit einer erkannten Sicherheitsausrüstung und/oder einer geeigneten Arbeitskleidung Einstellungen zu einer Steuerung und/oder Regelung der Antriebseinheit und/oder von Sicherheitsfunktionen der Werkzeugmaschine über die Kommunikationseinheit an die Steuer- und/oder Regeleinheit überträgt.

Des Weiteren kann es zweckmäßig sein, dass die Werkzeugmaschine zumindest eine Informationsausgabeeinheit aufweist, welche dazu eingerichtet ist, den Bediener der Werkzeugmaschine in Abhängigkeit der sensierten Daten, insbesondere mittels eines optischen, eines haptischen und/oder eines akustischen Signals, zu informieren. Eine Informationsausgabeeinheit zu einer Informationsausgabe an einen Bediener ist vorzugsweise als optische, akustische und/oder haptische Informationsausgabeeinheit ausgebildet. Hierbei ist die Informationsausgabeeinheit vorzugsweise ein Bestandteil der Werkzeugmaschine. Es ist jedoch auch denkbar, dass die Informationsausgabeeinheit ein Bestandteil einer externen Einheit, wie beispielsweise eines Smartphones, eines Tablets, eines PCs, eines Laptops o. dgl., ist. Die Informationsausgabeeinheit umfasst zu einer Informationsausgabe an einen Bediener vorzugsweise zumindest eine optische Ausgabeeinheit, wie beispielsweise ein LC-Display, ein berührungsempfindliches Display, ein LED-Display, ein Plasma-Display o. dgl., zu einer optischen Informationsausgabe an einen Bediener. Bevorzugt umfasst die Informationsausgabeeinheit zumindest eine akustische Ausgabeeinheit, wie beispielsweise einen Lautsprecher o. dgl., zu einer akustischen Informationsausgabe an einen Bediener. Besonders bevorzugt umfasst die Informationsausgabeeinheit zumindest eine haptische Ausgabeeinheit, wie beispielsweise eine Schwingungserregereinheit o. dgl., zu einer haptischen Informationsausgabe an einen Bediener. Es ist jedoch auch denkbar, dass eine Informationsausgabe an einen Bediener infolge einer Ansteuerung der Antriebseinheit mittels der Steuer- und/oder Regeleinheit erfolgt. Hierbei ist es denkbar, dass eine Informationsausgabe an einen Bediener beispielsweise durch eine Drehzahlschwankung einer Antriebseinheitsdrehzahl o. dgl. erfolgt. Weitere, einem Fachmann als sinnvoll erscheinende antriebseinheitsbezogene Informationsausgaben an einen Bediener sind ebenfalls denkbar.

Die Erfindung bezieht sich ferner auf eine Gartenwerkzeugmaschine, insbesondere einen Rasenmäher.

Die Erfindung bezieht sich weiter auf eine Handwerkzeugmaschine, insbesondere eine Bohrwerkzeugmaschine, eine Schraubendrehwerkzeugmaschine, eine Schleifwerkzeugmaschine oder eine Trennwerkzeugmaschine.

Die Erfindung bezieht sich auch auf ein System aufweisend zumindest eine Werkzeugmaschine mit zumindest einer von der Sensoreinheit sensierbaren Zubehöreinrichtung.

### Kurze Beschreibung der Zeichnungen

Weitere Vorteile ergeben sich aus der folgenden Zeichnungsbeschreibung. In der Zeichnung sind Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Hierbei zeigt:
- Fig. 1: eine schematische Darstellung einer ersten Ausführungsform einer erfindungsgemäßen Werkzeugmaschine,
- Fig. 2: eine schematische Darstellung einer zweiten Ausführungsform einer erfindungsgemäßen Werkzeugmaschine und
- Fig. 3: eine schematische Darstellung einer dritten Ausführungsform einer erfindungsgemäßen Werkzeugmaschine.

In den folgenden Figuren sind gleiche Bauteile mit den gleichen Bezugszeichen versehen.

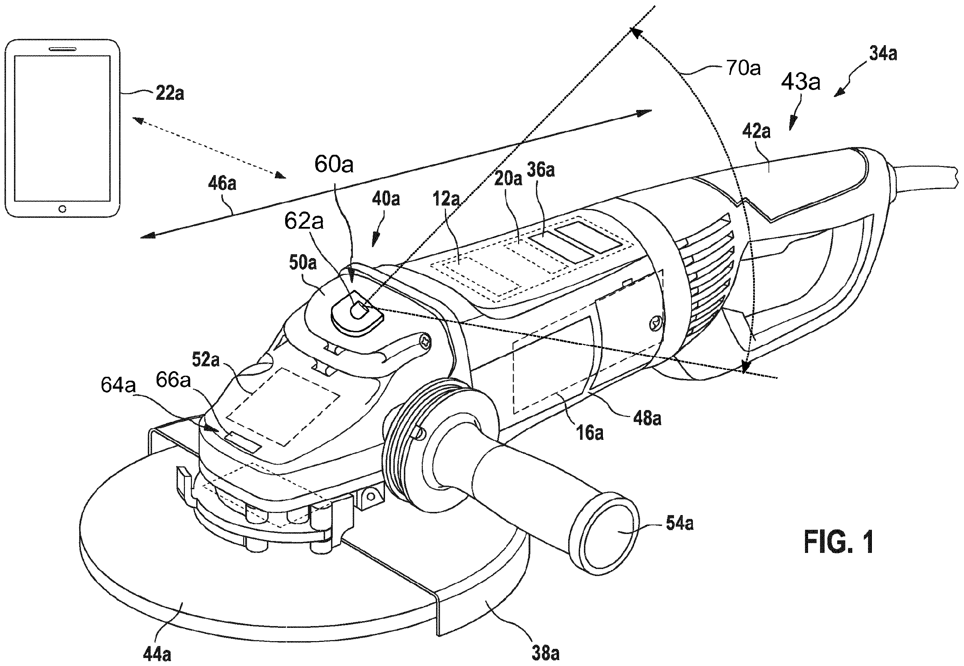

Figur 1 zeigt eine tragbare Werkzeugmaschine 34a. Hierbei ist die Werkzeugmaschine 34a als Winkelschleifer ausgebildet. Die Werkzeugmaschine 34a umfasst zumindest eine als Schutzhaube ausgebildete Zubehörvorrichtung 38a. Ferner umfasst die Werkzeugmaschine 34a zumindest ein Werkzeugmaschinengehäuse 40a und einen Haupthandgriff 42a, der sich an einer einem Einsatzwerkzeug 44a abgewandten Seite des Werkzeugmaschinengehäuses 40a in Richtung einer Haupterstreckungsrichtung 46a der Werkzeugmaschine 34a erstreckt. Das Einsatzwerkzeug 44a ist hierbei als Schleifscheibe ausgebildet. Es ist jedoch auch denkbar, dass das Einsatzwerkzeug 44a als Trenn- oder Polierscheibe ausgebildet ist. Das Werkzeugmaschinengehäuse 40a umfasst ein Motorgehäuse 48a zu einer Aufnahme einer Antriebseinheit 16a der Werkzeugmaschine 34a. Ferner umfasst das Werkzeugmaschinengehäuse 40a ein Getriebegehäuse 50a zu einer Aufnahme einer Abtriebseinheit 52a der Werkzeugmaschine 34a. Die Antriebseinheit 16a ist dazu vorgesehen, das Einsatzwerkzeug 44a über die Abtriebseinheit 52a rotierend anzutreiben. An dem Getriebegehäuse 50a ist eine weitere als Zusatzhandgriffeinheit ausgebildete Zubehörvorrichtung 54a angeordnet. Die als Zusatzhandgriffeinheit ausgebildete Zubehörvorrichtung 54a erstreckt sich quer zur Haupterstreckungsrichtung 46a der Werkzeugmaschine 34a.

Die Werkzeugmaschine 34a umfasst vorzugsweise eine netzbetriebene Energieversorgungsvorrichtung. Alternativ oder zusäztlich kann die Werkzeugmaschine 34a unabhängig von einem Netzbetrieb mittels einer Akkupack betreibbar sein.

Die Werkzeugmaschine 34a umfasst ferner zumindest eine Steuer- oder Regeleinheit 12a zur Steuerung oder Regelung der Antriebseinheit 16a.

Die Werkzeugmaschine 34a weist zumindest eine erste Sensoreinheit 60a mit einem ersten Bildsensor 62a auf, welcher dazu eingerichtet ist, bedienerspezifische Daten des Bediener zu sensieren und mittels einer Auswerteeinheit (nicht gezeigt) auszuwerten. In Abhängigkeit der sensierten und ausgewerteten Daten wird die Antriebseinheit 16a mittels der Steuer- oder Regeleinheit 12a gesteuert oder geregelt.

Der erste Bildsensor 62a ist dazu eingerichtet, Vitaldaten, insbesondere eine Herzfrequenz und/oder einen Ermüdungszustand, eines Bedieners zu erfassen. Dabei kann beispielsweise erfasst werden, ob der Bediener eine ausreichend hohe Herzfrequenz hat und/oder in wieweit die Herzfrequenz sich im Betrieb der Werkzeugmaschine ändert insbesondere erhöht oder verringert.

Der erste Bildsensor 62a ist dazu eingerichtet, zumindest eine als Schutzbrille und/oder als Handschuh und/oder als Sicherheitskleidung ausgebildete Zubehöreinrichtung des Bedieners zu erfassen. Alternativ oder zusätzlich kann der erste Bildsensor 62a dazu eingerichtet sein, eine als Sicherheitsschuh ausgebildete Zubehörvorrichtung zu erfassen. Mittels des ersten Bildsensors 62a kann überprüft werden, ob der Bediener die Zubehöreinrichtung trägt.

Die erste Sensoreinheit 60a und/oder der erste Bildsensor 62a sind dabei derart an einer von dem Einsatzwerkzeug 44a abgewandten Seite des Werkzeugmaschinengehäuses 40a angeordnet, um die Zubehöreinrichtung/en des Bedieners in zumindest einem Arbeitsschritt vor und/oder während des Betriebszustands der Antriebseinheit 16a zu sensieren.

Der erste Bildsensor 62a ist dazu eingerichtet, zumindest ein Auge oder eine Lidbewegung eines Auges des Bedieners zu überwachen, um einen Ermüdungszustand oder eine Verletzung des Auges zu erkennen.

Der erste Bildsensor 62a ist dazu eingerichtet, eine Schwenkbewegung bzw. eine Verdrehbewegung des Kopfs des Bedieners zu sensieren. Beispielsweise kann ein Augenabstand beider Augen des Bedieners oder eine Formänderung zumindest einer Augenkontur eines, insbesondere jedes, Auges des Bedieners erfasst werden. Hierdurch kann mittels einer Auswerteeinheit ausgewertet werden, ob ein Blickwinkel des Bedieners auf den Arbeitsbereich gerichtet ist.

Der erste Bildsensor 62a ist dazu eingerichtet, ein Gesicht des Bedieners zu sensieren und zu erkennen, ob der Bediener handhabungsberechtigt ist. Dabei wird ein Gesicht oder einzelne markante Gesichtspartien des Bedieners mittels des ersten Bildsensors 62a sensiert und in einer Speichereinheit (nicht dargestellt) abgespeichert. Bei einem Betrieb der Werkzeugmaschine kann der erste Bildsensor 62a das Gesicht oder einzelne Gesichtspartien des Bedieners sensieren und mittels der gespeicherten Daten abgleichen, um eine Bedienung der Werkzeugmaschine 34a im Falle eines handhabungsberechtigten Bedieners freizugeben oder im Falle eines nicht handhabungsberechtigten Bedieners zu sperren.

Der Bildsensor 62a ist ferner dazu eingerichtet, einen Arbeitsbereich zu beobachten, um die Antriebseinheit 12a in Abhängigkeit eines sich in dem Arbeitsbereich befindlichen Körperteils eines Bedieners oder einer sich in dem Arbeitsbereich befindlichen Person zu steuern oder zu regeln. Dabei kann der Bildsensor 62a derart an der Werkzeugmaschine 34a angeordnet sein, dass sich, vorzugsweise mit Verwendung einer entsprechenden Objektiveinheit, beispielsweise einen 360° Blickwinkel ergibt.

Der erste Bildsensor 62a ist dazu eingerichtet, einen Istzustand der Werkzeugmaschine 34a in zumindest einem Arbeitsschritt vor und während des Betriebszustands der Antriebseinheit 16a zu sensieren und mit einem Sollzustand abzugleichen und in Abhängigkeit einer Abweichung vom Istzustand gegenüber dem Sollzustand die Antriebseinheit 16a zu steuern oder zu regeln. Dabei sensiert der erste Bildsensor 62a bedienerspezifische Daten in zumindest einem Arbeitsschritt vor und/oder während des Betriebszustands der Antriebseinheit 16a.

Der erste Bildsensor 62a ist dazu eingerichtet, zu erkennen, ob der Bediener die Werkzeugmaschine 34a betätigt, indem zumindest ein Bereich eines Blickwinkels 70a des ersten Bildsensors 62a auf den Griffbereich 43a des Haupthandgriffs 42a der Werkzeugmaschine gerichtet ist. Dadurch kann sensiert werden, ob eine Hand des Bedieners den Griffbereich 43a umgreift.

Die Werkzeugmaschine 34a weist eine zweite Sensoreinheit 64a mit einem zweiten Bildsensor 66a auf. Der zweite Bildsensor 66a ist zu dem ersten Bildsensor 62a beabstandet. Der erste Bildsensor 62a ist im Wesentlichen auf den Bediener der Werkzeugmaschine 34a ausgerichtet. Der zweite Bildsensor 66a ist auf den Arbeitsbereich der Werkzeugmaschine 34a ausgerichtet. Durch einen auf den Arbeitsbereich gerichteten zweiten Bildsensor 66a kann erkannt werden, welches Einsatzwerkzeug 44a verwendet wird und/oder ob beispielsweise die als Schutzhaube ausgebildete Zubehörvorrichtung 38a der Werkzeugmaschine 34a verwendet wird.

Der erste Bildsensor 62a sensiert vorzugsweise Daten im Infrarotstrahlungsbereich, wodurch Gefahrenquellen wie beispielsweise Menschen oder Tiere besonders einfach und zuverlässig erkannt werden.

Die Werkzeugmaschine 34a weist eine Kommunikationseinheit 20a zu einer Kommunikation mit einer externen Einheit 22a zu einem Austausch von elektronischen Daten zu einer Steuerung oder Regelung der Antriebseinheit 16a auf. Die Kommunikationseinheit 20a ist vorzugsweise als kabellose Kommunikationseinheit 20a ausgebildet. Zudem sind elektronische Daten mittels der Kommunikationseinheit 20a an die externe Einheit 22a übertragbar.

Die externe Einheit 22a ist vorzugsweise als Smartphone ausgebildet, das eine App zu einer Kommunikation mit der Kommunikationseinheit 20a aufweist.

Die Werkzeugmaschine 34a weist zumindest eine Informationsausgabeeinheit 36a auf, welche dazu eingerichtet ist, den Bediener der Werkzeugmaschine 34a in Abhängigkeit der sensierten Daten, insbesondere mittels eines optischen, eines haptischen und/oder eines akustischen Signals, zu informieren.

Ferner gibt die Steuer- und/oder Regeleinheit 12a in Abhängigkeit von werkzeugmaschinensensorerfassten Daten und/oder kommunikationseinheitsübermittelten Daten zumindest eine Information mittels der Informationsausgabeeinheit 36a der Werkzeugmaschine 34a aus, insbesondere zu einer Information des Bedieners über einen Werkzeugmaschinenzustand und/oder zu einer Warnung vor einer Gefährdung. Zudem steuert und/oder regelt die Steuer- oder Regeleinheit 12a zumindest eine Betriebsmoduseinstellung der Werkzeugmaschine 34a in Abhängigkeit von kommunikationseinheitsübermittelten Daten.

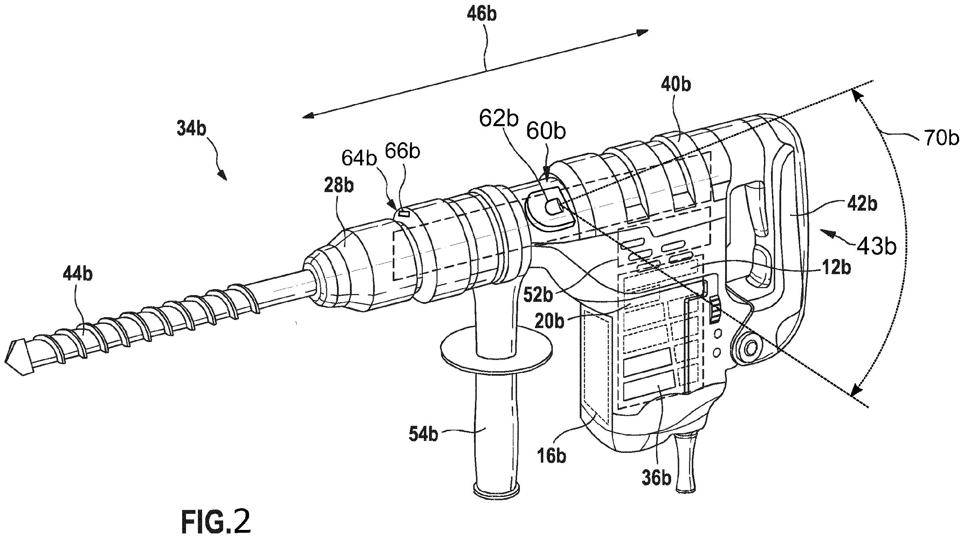

Figuren 2 bis 3 zeigen weitere Ausführungsbeispiele der Erfindung. Die nachfolgende Beschreibung und die Zeichnung beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnung und/oder die Beschreibung der anderen Ausführungsbeispiele, insbesondere der Figur 1, verwiesen werden kann. Zur Unterscheidung der Ausführungsbeispiele ist der Buchstabe a den Bezugszeichen des Ausführungsbeispiels in der Figur 1 nachgestellt. In den Ausführungsbeispielen der Figuren 2 bis 3 ist der Buchstabe a durch den Buchstaben b oder d ersetzt.

Figur 2 zeigt eine als als Bohr- und/oder Meißelhammer ausgebildete Werkzeugmaschine 34b. Die Werkzeugmaschine 34b umfasst zumindest eine Schlagwerkvorrichtung (nicht gezeigt). Ferner umfasst die Werkzeugmaschine 34b ein Werkzeugmaschinengehäuse 40b, an dem in einem Frontbereich eine Werkzeugaufnahme 28b der Werkzeugmaschine 34b zu einer Aufnahme eines Einsatzwerkzeugs 44b angeordnet ist. An einer dem Frontbereich abgewandten Seite umfasst die Werkzeugmaschine 34b einen Haupthandgriff 42b zu einer Führung der Werkzeugmaschine 34b und zu einer Übertragung einer Kraft, insbesondere einer Andrückkraft, von einem Bediener auf die Werkzeugmaschine 34b. Die Werkzeugmaschine 34b ist ferner mit einer lösbaren Zusatzhandgriffeinheit ausgeführt. Hierbei kann die Zusatzhandgriffeinheit über eine Rastverbindung oder andere, einem Fachmann als sinnvoll erscheinende Verbindungen lösbar am Werkzeugmaschinengehäuse 40b befestigt sein.

Die erste Sensoreinheit 60b und/oder der erste Bildsensor 62b sind an einer Seitenfläche des Werkzeugmaschinengehäuses 40b angeordnet und zumindest teilweise an einer von dem Einsatzwerkzeug 44b abgewandten Seite des Werkzeugmaschinengehäuses 40b angeordnet, um die Zubehöreinrichtung/en des Bedieners in zumindest einem Arbeitsschritt vor und/oder während des Betriebszustands der Antriebseinheit 16b zu sensieren.

Die Werkzeugmaschine 34b weist eine zweite Sensoreinheit 64b mit einem zweiten Bildsensor 66b auf. Der zweite Bildsensor 66b ist zu dem ersten Bildsensor 62b beabstandet. Der erste Bildsensor 62b ist im Wesentlichen auf den Bediener der Werkzeugmaschine 34b ausgerichtet. Der zweite Bildsensor 66b ist auf den Arbeitsbereich der Werkzeugmaschine 34b ausgerichtet. Durch einen auf den Arbeitsbereich gerichteten zweiten Bildsensor 66b kann erkannt werden, welches Einsatzwerkzeug 44b verwendet wird und/oder ob beispielsweise die als Schutzhaube ausgebildete Zubehörvorrichtung 38b der Werkzeugmaschine 34b verwendet wird.

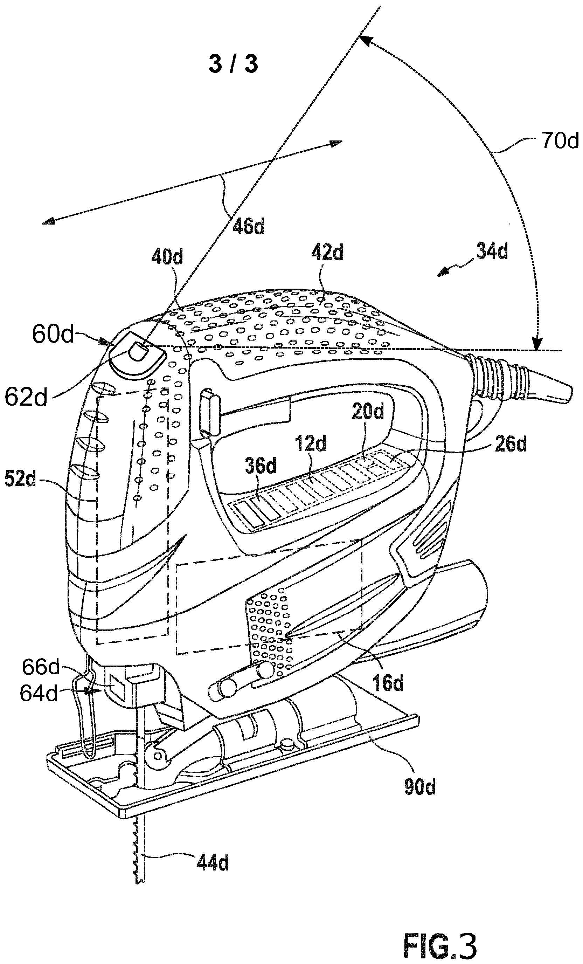

Figur 3 zeigt eine als Stichsäge ausgebildete Werkzeugmaschine 34d. Die Werkzeugmaschine 34d weist ein Werkzeugmaschinengehäuse 40d auf, das eine Antriebseinheit 16d der Werkzeugmaschine 34d und eine Abtriebseinheit 52d der Werkzeugmaschine 34d umschließt. Die Antriebseinheit 16d und die Abtriebseinheit 52d sind dazu vorgesehen, ein in einer Werkzeugaufnahme (nicht gezeigt) der Werkzeugmaschine 34d eingespanntes Einsatzwerkzeug 44d oszillierend anzutreiben. Hierbei wird das Einsatzwerkzeug 44d im Wesentlichen senkrecht zu einer Bearbeitungsrichtung oszillierend angetrieben. Das Einsatzwerkzeug 44d ist als Stichsägeblatt ausgebildet. Ein oszillierender Antrieb des Einsatzwerkzeugs 44d erfolgt hierbei auf eine, einem Fachmann bereits bekannte Art und Weise.

Die erste Sensoreinheit 60d und/oder der erste Bildsensor 62d sind derart an einer von dem Einsatzwerkzeug 44d abgewandten Seite des Werkzeugmaschinengehäuses 40d angeordnet, um die Zubehöreinrichtung/en des Bedieners in zumindest einem Arbeitsschritt vor und/oder während des Betriebszustands der Antriebseinheit 16d zu sensieren.

Die Werkzeugmaschine 34d weist eine zweite Sensoreinheit 64d mit einem zweiten Bildsensor 66d auf. Der zweite Bildsensor 66d ist zu dem ersten Bildsensor 62d beabstandet. Der erste Bildsensor 62d ist im Wesentlichen auf den Bediener der Werkzeugmaschine 34d ausgerichtet. Der zweite Bildsensor 66d ist auf den Arbeitsbereich der Werkzeugmaschine 34d ausgerichtet. Durch einen auf den Arbeitsbereich gerichteten zweiten Bildsensor 66d kann erkannt werden, welches Einsatzwerkzeug 44d verwendet wird und/oder ob beispielsweise die als Schutzhaube ausgebildete Zubehörvorrichtung 38d der Werkzeugmaschine 34d verwendet wird.

## Patentansprüche

1. Handwerkzeugmaschine (34a, 34b, 34d) mit einer Antriebseinheit (16a, 16b, 16d) zum Antrieb eines mit der Handwerkzeugmaschine (34a, 34b, 34d) betreibbar aufnehmbaren Einsatzwerkzeugs (44a, 44b, 44d) und mit einer Steuer- oder Regeleinheit (12a, 12b, 12d) zur Steuerung oder Regelung der Antriebseinheit (16a, 16b, 16d), **dadurch gekennzeichnet, dass** eine erste Sensoreinheit (60a, 60b, 60d) einen ersten Bildsensor (62a, 62b, 62d)-aufweist, der auf einen Bediener der Handwerkzeugmaschine (34a, 34b, 34d) ausgerichtet ist, und dass die erste Sensoreinheit (60a, 60b, 60d) dazu eingerichtet ist, Vitaldaten, insbesondere eine Herzfrequenz und/oder einen Ermüdungszustand, oder eine Zubehöreinrichtung, insbesondere eine Schutzbrille, des Bedieners zu erfassen und mittels der Steuer- oder Regeleinheit (12a, 12b, 12d) derart zu verarbeiten, dass die Antriebseinheit (16a, 16b, 16d) in Abhängigkeit der erfassten Daten gesteuert oder geregelt wird, und dass zumindest eine zweite Sensoreinheit (64a, 64b, 64d), welche zu der ersten Sensoreinheit (60a, 60b, 60d) beabstandet ist, auf den Arbeitsbereich ausgerichtet ist.

2. Handwerkzeugmaschine (34a, 34b, 34d) nach Anspruch 1, **dadurch gekennzeichnet, dass** die erste Sensoreinheit (60a, 60b, 60d) dazu eingerichtet ist, zumindest ein Auge oder eine Lidbewegung eines Auges des Bedieners zu überwachen um einen Ermüdungszustand oder eine Verletzung des Auges zu erkennen.

3. Handwerkzeugmaschine (34a, 34b, 34d) nach einem der vorhergehenden Ansprüche, **dadurch gekennzeichnet, dass** die erste Sensoreinheit (60a, 60b, 60d) dazu eingerichtet ist, ein Gesicht des Bedieners zu sensieren und insbesondere zu erkennen, ob der Bediener handhabungsberechtigt ist.

4. Handwerkzeugmaschine (34a, 34b, 34d) nach einem der vorhergehenden Ansprüche, **dadurch gekennzeichnet, dass** die erste Sensoreinheit (60a, 60b, 60d) dazu eingerichtet ist, einen Istzustand der Werkzeugmaschine (34a, 34b, 34d) in zumindest einem Arbeitsschritt vor und/oder während des Betriebszustands der Antriebseinheit (16a, 16b, 16d) zu sensieren und mit einem Sollzustand abzugleichen und in Abhängigkeit einer Abweichung vom Istzustand gegenüber dem Sollzustand die Antriebseinheit (16a, 16b, 16d) zu steuern oder zu regeln.

5. Handwerkzeugmaschine (34a, 34b, 34d) nach Anspruch 4, **dadurch gekennzeichnet, dass** die erste Sensoreinheit (60a, 60b, 60d) dazu eingerichtet ist, zu erkennen, ob der Bediener die Werkzeugmaschine (34a, 34b, 34d) betätigt.

6. Handwerkzeugmaschine (34a, 34b, 34d) nach einem der vorhergehenden Ansprüche, **dadurch gekennzeichnet, dass** die erste und/oder die zweite Sensoreinheit (64a, 64b, 64d) Daten im Infrarotstrahlungsbereich sensiert.

7. Handwerkzeugmaschine (34a, 34b, 34d) nach einem der vorhergehenden Ansprüche, **gekennzeichnet durch** zumindest eine Kommunikationseinheit (20a, 20b, 20d) zu einer Kommunikation mit zumindest einer externen Einheit (22a) zu einem Austausch von elektronischen Daten zumindest zu einer Steuerung oder Regelung der Antriebseinheit (16a, 16b, 16d).

8. Handwerkzeugmaschine (34a, 34b, 34d) nach einem der vorhergehenden Ansprüche, **gekennzeichnet durch** zumindest eine Informationsausgabeeinheit (36a, 36b, 36d), welche dazu eingerichtet ist, den Bediener der Handwerkzeugmaschine (34a, 34b, 34d) in Abhängigkeit der sensierten Daten, insbesondere mittels eines optischen, eines haptischen und/oder eines akustischen Signals, zu informieren.

## Claims

1. Hand-held power tool (34a, 34b, 34d), having a drive unit (16a, 16b, 16d) for driving a receivable insert tool (44a, 44b, 44d) that can be operated by means of the hand-held power tool (34a, 34b, 34d), and having an open-loop or closed-loop control unit (12a, 12b, 12d) for controlling the drive unit (16a, 16b, 16d) by open-loop or closed-loop control, **characterized in that** a first sensor unit (60a, 60b, 60d) has a first image sensor (62a, 62b, 62d) which is oriented toward an operator of the hand-held power tool (34a, 34b, 34d), and **in that** the first sensor unit (60a, 60b, 60d) is configured to sense vital data, in particular a heart rate and/or a state of fatigue, or an accessory means, in particular safety eyewear, of the operator and to process same by means of the open-loop or closed-loop control unit (12a, 12b, 12d) in such a manner that the drive unit (16a, 16b, 16d) is controlled, by open-loop or closed-loop control, in dependence on the sensed data, and **in that** at least one second sensor unit (64a, 64b, 64d), which is spaced apart from the first sensor unit (60a, 60b, 60d), is oriented toward the work area.

2. Hand-held power tool (34a, 34b, 34d) according to Claim 1, **characterized in that** the first sensor unit (60a, 60b, 60d) is configured to monitor at least one eye, or a lid movement of an eye, of the operator, in order to identify a state of fatigue or injury to the eye.

3. Hand-held power tool (34a, 34b, 34d) according to either of the preceding claims, **characterized in that** the first sensor unit (60a, 60b, 60d) is configured to sense a face of the operator, and in particular to identify whether the operator is authorized to operate.

4. Hand-held power tool (34a, 34b, 34d) according to one of the preceding claims, **characterized in that** the first sensor unit (60a, 60b, 60d) is configured to sense an actual state of the power tool (34a, 34b, 34d), in at least one work step, before and/or while the drive unit (16a, 16b, 16d) is in the operating state, and to compare it with a reference state, and to control the drive unit (16a, 16b, 16d), by open-loop or closed-loop control, in dependence on a deviation by the actual state in comparison with the reference state.

5. Hand-held power tool (34a, 34b, 34d) according to Claim 4, **characterized in that** the first sensor unit (60a, 60b, 60d) is configured to identify whether the operator is operating the power tool (34a, 34b, 34d).

6. Hand-held power tool (34a, 34b, 34d) according to one of the preceding claims, **characterized in that** the first and/or the second sensor unit (64a, 64b, 64d) senses data in the infrared radiation range.

7. Hand-held power tool (34a, 34b, 34d) according to one of the preceding claims, **characterized by** at least one communication unit (20a, 20b, 20d) for communicating with at least one external unit (22a), for the purpose of exchanging electronic data, at least for the purpose of controlling the drive unit (16a, 16b, 16d) by open-loop or closed-loop control.

8. Hand-held power tool (34a, 34b, 34d) according to one of the preceding claims, **characterized by** at least one information output unit (36a, 36b, 36d), which is configured to provide information to the operator of the hand-held power tool (34a, 34b, 34d) in dependence on the sensed data, in particular by means of an optical, haptic and/or acoustic signal.

## Revendications

1. Machine-outil à main (34a, 34b, 34d) comprenant une unité d'entraînement (16a, 16b, 16d) destinée à entraîner un outil d'insertion (44a, 44b, 44d) qui peut être reçu de manière à pouvoir être utilisé avec la machine-outil à main (34a, 34b, 34d) et une unité de commande ou de régulation (12a, 12b, 12d) destinée à commander ou réguler l'unité d'entraînement (16a, 16b, 16d), **caractérisée en ce qu'**une première unité de détection (60a, 60b, 60d) comporte un premier capteur d'image (62a, 62b, 62d) qui est orienté vers un utilisateur de la machine-outil à main (34a, 34b, 34d) et **en ce que** la première unité de détection (60a, 60b, 60d) est adaptée pour détecter des données vitales, en particulier une fréquence cardiaque et/ou un état de fatigue, ou un dispositif accessoire, en particulier des lunettes de protection, de l'utilisateur et pour les traiter au moyen de l'unité de commande ou de régulation (12a, 12b, 12d) de façon à commander ou réguler l'unité d'entraînement (16a, 16b, 16d) en fonction des données détectées, et **en ce qu'**au moins une deuxième unité de détection (64a, 64b, 64d), qui est à une certaine distance de la première unité de détection (60a, 60b, 60d), est orientée vers la zone de travail.

2. Machine-outil à main (34a, 34b, 34d) selon la revendication 1, **caractérisée en ce que** la première unité de détection (60a, 60b, 60d) est adaptée pour surveiller au moins un œil ou un mouvement de paupière d'un œil de l'utilisateur pour détecter un état de fatigue ou une blessure de l'œil.

3. Machine-outil à main (34a, 34b, 34d) selon l'une des revendications précédentes, **caractérisée en ce que** la première unité de détection (60a, 60b, 60d) est adaptée pour capter le visage de l'utilisateur et notamment pour détecter si l'utilisateur est autorisé à utiliser la machine-outil.

4. Machine-outil à main (34a, 34b, 34d) selon l'une des revendications précédentes, **caractérisée en ce que** la première unité de détection (60a, 60b, 60d) est adaptée pour capter un état réel de la machine-outil (34a, 34b, 34d) dans au moins une étape de travail et/ou pendant l'état de fonctionnement de l'unité d'entraînement (16a, 16b, 16d) et pour le comparer à un état de consigne et commander ou réguler l'unité d'entraînement (16a, 16b, 16d) en fonction d'un écart de l'état réel par rapport à l'état de consigne.

5. Machine-outil à main (34a, 34b, 34d) selon la revendication 4, **caractérisée en ce que** la première unité de détection (60a, 60b, 60d) est adaptée pour détecter si l'utilisateur actionne la machine-outil (34a, 34b, 34d).

6. Machine-outil à main (34a, 34b, 34d) selon l'une des revendications précédentes, **caractérisée en ce que** la première et/ou la deuxième unité de détection (64a, 64b, 64d) capte(nt) des données dans le domaine du rayonnement infrarouge.

7. Machine-outil à main (34a, 34b, 34d) selon l'une des revendications précédentes, **caractérisée par** au moins une unité de communication (20a, 20b, 20d) destinée à la communication avec au moins une unité extérieure (22a) pour effectuer un échange de données électroniques au moins pour commander ou réguler l'unité d'entraînement (16a, 16b, 16d).

8. Machine-outil à main (34a, 34b, 34d) selon l'une des revendications précédentes, **caractérisée par** au moins une unité de sortie d'informations (36a, 36b, 36d) qui est adaptée pour informer l'utilisateur de la machine-outil à main (34a, 34b, 34d) en fonction des données captées, notamment au moyen d'un signal optique, haptique et/ou acoustique.
